# EUROPEAN PATENT APPLICATION

(11) **EP 4 534 561 A1**
(43) Date of publication of application: **09.04.2025**
(21) Application number: 23810992.0
(22) Date of filing: 22.05.2023
(51) Int. Cl.: C07K 16/46, C07K 16/28, C07K 19/00, C12N 5/07, C12N 15/11, C12N 15/03, A61K 35/17, A61P 35/00, A61P 35/02

(54) **BISPECIFIC CHIMERIC ANTIGEN RECEPTOR TARGETING BCMA-CD19 AND APPLICATION THEREOF**

(30) Priority: 24.05.2022 CN 202210568686
(71) Applicant: Juventas Unicare Pharmaceutical (Beijing) Co., Ltd., Beijing 102206 (CN)
(72) Inventor: ZHANG, Chao, Beijing 102206 (CN); ZHANG, Qimeng, Beijing 102206 (CN); BAI, Dayong, Beijing 102206 (CN); LV, Lulu, Beijing 102206 (CN); ZHOU, Li, Beijing 102206 (CN); WANG, Yongzeng, Beijing 102206 (CN); ZHANG, Yunlong, Beijing 102206 (CN); DING, Wei, Beijing 102206 (CN); LU, Jiaxing, Beijing 102206 (CN)
(74) Representative: Ponti & Partners, S.L.P
(86) International application number: PCT/CN2023/095470
(87) International publication number: WO 2023/226921

(57) **Abstract**

The present invention provides a bispecific chimeric antigen receptor targeting BCMA-CD19 and an application thereof. The bispecific chimeric antigen receptor comprises an extracellular antigen recognition domain; the extracellular antigen recognition domain comprises an anti-BCMA extracellular antigen recognition domain and an anti-CD19 extracellular antigen recognition domain; the anti-BCMA extracellular antigen recognition domain comprises BCMA VH and BCMA VL, wherein amino acid sequences of BCMA VH complementarity-determining regions CDR1, CDR2 and CDR3 respectively comprise amino acid sequences as represented by SEQ ID NO: 1, SEQ ID NO: 2 and SEQ ID NO: 3, and amino acid sequences of BCMA VL complementarity-determining regions CDR1, CDR2 and CDR3 comprise amino acid sequences as represented by SEQ ID NO: 4, SEQ ID NO: 5 and SEQ ID NO: 6.

## Description

### TECHNICAL FIELD

The present application relates to the field of biomedicine, specifically to a bispecific chimeric antigen receptor targeting BCMA-CD19 and application thereof.

### BACKGROUND

Multiple myeloma, defined as a malignant proliferation of plasma cells in the bone marrow, is the second most common hematological malignancy, accounting for 1% of all cancer types. Studies have shown that multiple myeloma is more common among people over 60 years old and its incidence rate has increased steadily in recent years. For most patients, multiple myeloma is incurable and eventually develops into relapsed/refractory multiple myeloma. The survival time of patients with relapsed/refractory multiple myeloma who are ineffective against existing multiple myeloma treatments (such as immunomodulators, proteasome inhibitors, and antibody drugs) is only about 13 months.

B Cell Maturation Antigen (BCMA) is a transmembrane glycoprotein that belongs to the tumor necrosis factor receptor family. BCMA is highly expressed in multiple myeloma cells and not in most other cells. Malignant tumor plasma cells usually express higher levels of BCMA than those of normal plasma cells, the upregulation of BCMA promotes the growth of multiple myeloma cancer cells, while the downregulation of its expression can inhibit the growth of multiple myeloma cancer cells.

In addition, multiple myeloma, as a B cell line tumor, generally does not express the CD19 molecule, so CD19 is generally not a target for multiple myeloma therapy. However, some literatures suggest that some minor drug-resistant and relapsed multiple myeloma clones also have CD19⁺ phenotype.

Chimeric Antigen Receptor (CAR) is the core component of CAR cell therapy drugs, which can include a targeting portion (for example, a portion that binds to Tumor-Associated Antigen (TAA)), a hinge region, a transmembrane region and an intracellular domain. CAR-T cell immunotherapy is considered to be one of the most promising methods to conquer tumors. CAR-T cells use genetic modification methods to make T cells express CAR proteins, and such CAR protein has the ability to recognize intact proteins on the membrane surface without depending on antigen presentation, thereby causing T cell activation and functional effects.

In 2021, Bristol-Myers Squibb and Bluebird Bio announced together that the U.S. Food and Drug Administration (FDA) has approved their CAR-T cell therapy targeting BCMA (bb2121), which are used for adult patients with relapsed or refractory multiple myeloma after 4-line treatment (including immunomodulators, proteasome inhibitors, and antibody drug therapy). This is the world's first CAR-T cell therapy targeting BCMA. It is of practical significance to develop more effective cell therapies targeting BCMA.

### SUMMARY OF THE INVENTION

The present application provides a bispecific chimeric antigen receptor targeting BCMA-CD19 and application thereof. The inventors have constructed multiple expression vectors of bispecific chimeric antigen receptor targeting BCMA-CD19 and prepared bispecific CAR-T cells targeting BCMA-CD19. They also have verified that BCMA-CD19 bispecific CAR-T cells have good anti-tumor function at the cellular level.

A bispecific chimeric antigen receptor targeting BCMA-CD19, comprising an extracellular antigen recognition domain, a hinge region, a transmembrane region and an intracellular domain; wherein the extracellular antigen recognition domain comprises an anti-BCMA extracellular antigen recognition domain and an anti-CD19 extracellular antigen recognition domain;
the anti-BCMA extracellular antigen recognition domain comprises BCMA VH and BCMA VL, wherein amino acid sequences of BCMA VH complementarity-determining regions CDR1, CDR2 and CDR3 respectively comprise amino acid sequences as represented by SEQ ID NO: 1, SEQ ID NO: 2 and SEQ ID NO: 3, and amino acid sequences of BCMA VL complementarity-determining regions CDR1, CDR2 and CDR3 respectively comprise amino acid sequences as represented by SEQ ID NO: 4, SEQ ID NO: 5 and SEQ ID NO: 6.

In certain embodiments of the above-mentioned bispecific chimeric antigen receptor, the anti-CD19 extracellular antigen recognition domain comprises CD19 VH and CD19 VL, wherein amino acid sequences of CD19 VH complementarity-determining regions CDR1, CDR2 and CDR3 respectively comprise amino acid sequences as represented by SEQ ID NO: 7, SEQ ID NO: 8 and SEQ ID NO: 9, and amino acid sequences of CD19 VL complementarity-determining regions CDR1, CDR2 and CDR3 respectively comprise amino acid sequences as represented by SEQ ID NO: 10, SEQ ID NO: 11 and SEQ ID NO: 12.

In certain embodiments of the above-mentioned bispecific chimeric antigen receptor, the BCMA VH sequence comprises the amino acid sequence as represented by SEQ ID NO: 13, and the BCMA VL sequence comprises the amino acid sequence as represented by SEQ ID NO: 14.

In certain embodiments of the above-mentioned bispecific chimeric antigen receptor, the BCMA VH sequence comprises the amino acid sequence as represented by SEQ ID NO: 15, and the BCMA VL sequence comprises the amino acid sequence as represented by SEQ ID NO: 16.

In certain embodiments of the above-mentioned bispecific chimeric antigen receptor, the CD19 VH sequence comprises the amino acid sequence as represented by SEQ ID NO: 17, and the CD19 VL sequence comprises the amino acid sequence as represented by SEQ ID NO: 18.

In certain embodiments of the above-mentioned bispecific chimeric antigen receptor, the extracellular antigen recognition domain of the bispecific chimeric antigen receptor comprises any one structure selected from the group consisting of: CD19 VL sequence-1st linker sequence-CD19 VH sequence-2nd linker sequence-BCMA VL sequence-3rd linker sequence-BCMA VH sequence, BCMA VL sequence-4th linker sequence-BCMA VH sequence-5th linker sequence-CD19 VL sequence-6th linker sequence-CD19 VH sequence, BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence.

In certain embodiments of the above-mentioned bispecific chimeric antigen receptor, the extracellular antigen recognition domain of the bispecific chimeric antigen receptor comprises any one structure selected from the group consisting of: BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence.

In certain embodiments of the above-mentioned bispecific chimeric antigen receptor, the 1st linker sequence, the 2nd linker sequence, the 3rd linker sequence, the 4th linker sequence, the 5th linker sequence, the 6th linker sequence, the 7th linker sequence, the 8th linker sequence, the 9th linker sequence, the 10th linker sequence, the 11th linker sequence, and the 12th linker sequence are each independently selected from one or more of the following sequences: SEQ ID NO: 34, SEQ ID NO: 35, and SEQ ID NO: 36.

In certain embodiments of the above-mentioned bispecific chimeric antigen receptor, the extracellular antigen recognition domain of the bispecific chimeric antigen receptor comprises the amino acid sequence as represented by SEQ ID NO: 19 or SEQ ID NO: 20.

In certain embodiments of the above-mentioned bispecific chimeric antigen receptor, the hinge region is derived from one or more of IgG1, IgG4, CD4, CD7, CD28, CD84 and CD8α; optionally, the amino acid of the hinge region is derived from CD8α; further optionally, the amino acid sequence of the hinge region comprises the amino acid sequence as represented by SEQ ID NO: 21.

In certain embodiments of the above-mentioned bispecific chimeric antigen receptor, the transmembrane region is derived from one or more of CD3, CD4, CD7, CD8α, CD28, CD80, CD86, CD88, 4-1BB, CD152, OX40 and Fc70; optionally, the amino acid of the transmembrane region is derived from CD8α; further optionally, the amino acid sequence of the transmembrane region comprises the amino acid sequence as represented by SEQ ID NO: 22.

In certain embodiments of the above-mentioned bispecific chimeric antigen receptor, the intracellular domain comprises an intracellular signal transduction region; optionally, the intracellular domain further comprises a costimulatory signal transduction region.

In certain embodiments of the above-mentioned bispecific chimeric antigen receptor, the intracellular signal transduction region is derived from one or more of CD3ζ, CD3γ, CD3δ, CD3ε, CD5, CD22, CD79a, CD79b, FcRγ, FcRβ, CD66d, DAP10, DAP12 and Syk; optionally, the intracellular signal transduction region is derived from CD3ζ; further optionally, the amino acid sequence of the intracellular signal transduction region comprises the amino acid sequence as represented by SEQ ID NO: 23.

In certain embodiments of the above-mentioned bispecific chimeric antigen receptor, the costimulatory signal transduction region is derived from one of, two of or more of CD2, CD3, CD7, CD27, CD28, CD30, CD40, CD83, CD244, 4-1BB, OX40, LFA-1, ICOS, LIGHT, NKG2C, NKG2D, DAP10, B7-H3 and MyD88; optionally, the costimulatory signal transduction region is derived from CD28 and 4-1BB; further optionally, the amino acid sequence of the costimulatory signal transduction region comprises the amino acid sequence as represented by SEQ ID NO: 24.

In certain embodiments of the above-mentioned bispecific chimeric antigen receptor, the bispecific chimeric antigen receptor further comprises a guiding peptide located at the N-terminus of the amino acid sequence of the chimeric antigen receptor; optionally, the guiding peptide is derived from CD8α; further optionally, the amino acid sequence of the guiding peptide comprises the amino acid sequence as represented by SEQ ID NO: 25.

In certain embodiments of the above-mentioned bispecific chimeric antigen receptor, the bispecific chimeric antigen receptor comprises the amino acid sequence as represented by SEQ ID NO: 28 or SEQ ID NO: 29.

The present application also provides an isolated nucleic acid molecule comprising a nucleotide sequence encoding the above-mentioned bispecific chimeric antigen receptor.

In certain embodiments of the above-mentioned isolated nucleic acid molecule, the nucleotide sequence encoding the above-mentioned bispecific chimeric antigen receptor comprises:
1) a nucleotide sequence encoding a BCMA VH amino acid sequence as represented by SEQ ID NO: 13, which is represented by SEQ ID NO: 37; and a nucleotide sequence encoding a BCMA VL amino acid sequence as represented by SEQ ID NO: 14, which is represented by SEQ ID NO: 38; and/or
2) a nucleotide sequence encoding a CD19 VH amino acid sequence as represented by SEQ ID NO: 17, which is represented by SEQ ID NO: 39; and a nucleotide sequence encoding a CD19 VL amino acid sequence as represented by SEQ ID NO: 18, which is represented by SEQ ID NO: 40.

The present application also provides a vector comprising the above-mentioned isolated nucleic acid molecule.

In certain embodiments of the above-mentioned vector, the vector is an expression vector; in certain embodiments, the vector is a viral vector; in certain embodiments, the vector is a lentiviral vector.

The present application also provides an engineered immune effector cell comprising the above-mentioned chimeric antigen receptor, the above-mentioned isolated nucleic acid molecule or the above-mentioned vector.

In certain embodiments of the above-mentioned engineered immune effector cell, the engineered immune effector cell is selected from one or more of T lymphocyte, natural killer cell (NK cell), peripheral blood mononuclear cell (PBMC cell), pluripotent stem cell, T cell differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK) and embryonic stem cell.

In certain embodiments of the above-mentioned engineered immune effector cell, the engineered immune effector cell is T lymphocyte; optionally, the source of the T lymphocyte is autologous T lymphocyte or allogeneic T lymphocyte.

The present application also provides a pharmaceutical composition comprising the above-mentioned engineered immune effector cell, and pharmaceutically acceptable adjuvant.

In certain embodiments of the above-mentioned pharmaceutical composition, the pharmaceutically acceptable adjuvant includes a protective agent.

In certain embodiments of the above-mentioned pharmaceutical composition, the pharmaceutically acceptable adjuvant includes a cell cryopreservation solution.

In certain embodiments, the above-mentioned pharmaceutical composition is an intravenous injection.

The present application also provides use of the above-mentioned chimeric antigen receptor, the above-mentioned isolated nucleic acid molecule, the above-mentioned vector or the above-mentioned engineered immune effector cell in the preparation of a medicament, wherein the medicament is used for treating a disease or condition associated with the expression of BCMA.

In certain embodiments of the above-mentioned use, the disease or condition associated with the expression of BCMA is cancer; optionally, the cancer is multiple myeloma; further optionally, the cancer is refractory or relapsed multiple myeloma.

In certain embodiments of the above-mentioned use, the disease or condition associated with expression of BCMA may be an autoimmune disease.

In certain embodiments of the above-mentioned use, the autoimmune disease may be selected from the group consisting of systemic lupus erythematosus, rheumatoid arthritis, idiopathic thrombocytopenic purpura, myasthenia gravis, and autoimmune hemolytic anemia.

The present application also provides a method for treating a disease or condition associated with the expression of BCMA, including the following steps: administering an effective amount of the above-mentioned engineered immune effector cell or the above-mentioned pharmaceutical composition to a subject having a need to treat a disease or condition associated with the expression of BCMA.

In certain embodiments of the above-mentioned method, the disease or condition associated with the expression of BCMA is cancer; optionally, the cancer is multiple myeloma; further optionally, the cancer is refractory or relapsed multiple myeloma.

In certain embodiments of the above-mentioned method, the disease or condition associated with expression of BCMA may be an autoimmune disease.

In certain embodiments of the above-mentioned method, the autoimmune disease may be selected from the group consisting of systemic lupus erythematosus, rheumatoid arthritis, idiopathic thrombocytopenic purpura, myasthenia gravis, and autoimmune hemolytic anemia.

In certain embodiments of the above-mentioned method, the administration method is intravenous injection.

In certain embodiments of the above-mentioned method, the administration method is to administer an effective amount of the above-mentioned engineered immune effector cell or the above-mentioned pharmaceutical composition to a subject in a single injection.

In certain embodiments of the above-mentioned method, the effective amount of the above-mentioned engineered immune effector cell or the above-mentioned pharmaceutical composition is at a dose of 1×10⁵ to 1×10⁷ cells/kg.

The present application also provides the above-mentioned engineered immune effector cell or the above-mentioned pharmaceutical composition, for use in treating a disease or condition associated with the expression of BCMA.

In certain embodiments of the above-mentioned engineered immune effector cell or the above-mentioned pharmaceutical composition, the disease or condition associated with the expression of BCMA is cancer; optionally, the cancer is multiple myeloma; further optionally, the cancer is refractory or relapsed multiple myeloma.

In certain embodiments of the above-mentioned engineered immune effector cell or the above-mentioned pharmaceutical composition, the disease or condition associated with the expression of BCMA may be an autoimmune disease.

In certain embodiments of the above-mentioned engineered immune effector cell or the above-mentioned pharmaceutical composition, the autoimmune disease may be selected from the group consisting of systemic lupus erythematosus, rheumatoid arthritis, idiopathic thrombocytopenic purpura, myasthenia gravis, and autoimmune hemolytic anemia.

### BRIEF DESCRIPTION OF THE DRAWINGS

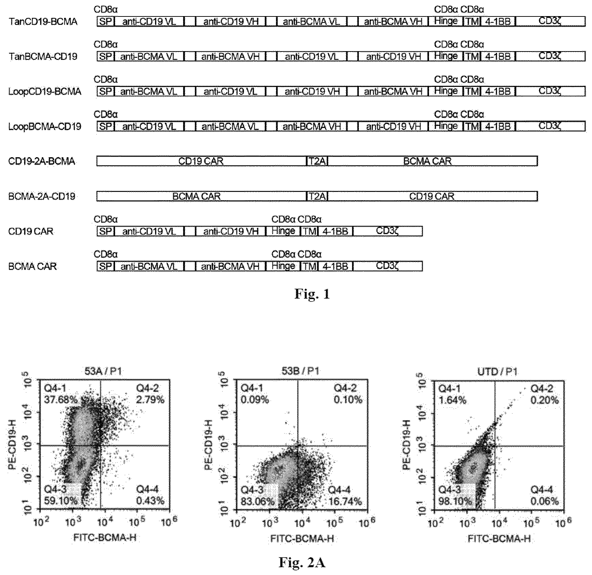

Fig. 1 shows a schematic structural diagram of various BCMA-CD19 bispecific CARs, CD19 CAR, and BCMA CAR in Example 1 of the present application.

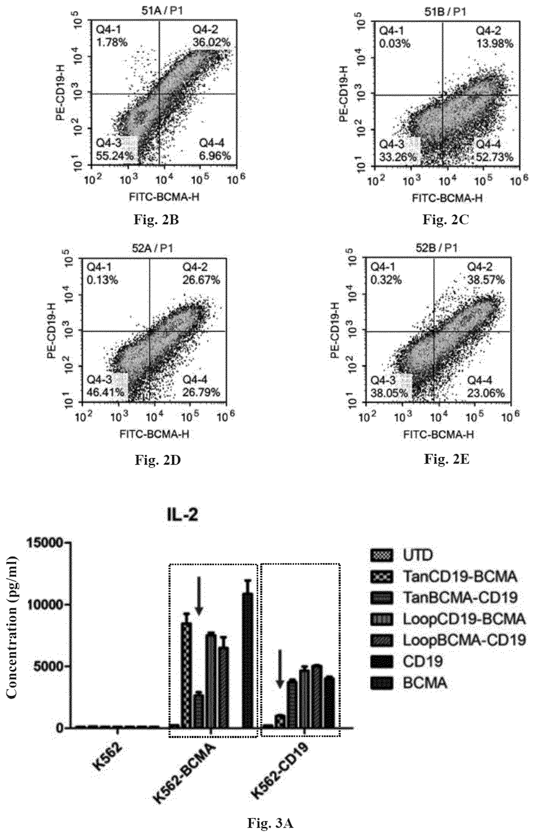

Fig. 2A shows the CAR expression of two intact CARs (i.e. CD19-2A-BCMA and BCMA-2A-CD19) linked by the self-cleaving polypeptide T2A on the surface of CAR-T cell (6 days after infection) in Example 2 of the present application; wherein the left figure of Fig. 2A is CD19-2A-BCMA group, the middle figure of Fig. 2A is BCMA-2A-CD19 group, and the right figure of Fig. 2A is UTD group (T cell without CAR transduction); Figs. 2B-2E show CAR expression on the surface of four bispecific CAR-T cells (i.e., Tan CD19-BCMA cells, Tan BCMA-CD19 cells, Loop CD19-BCMA cells, and Loop BCMA-CD19 cells) (6 days after infection) in Example 2 of the present application; wherein Fig. 2B is Tan CD19-BCMA group, Fig. 2C is Tan BCMA-CD19 group, Fig. 2D is Loop CD19-BCMA group, and Fig. 2E is Loop BCMA-CD19 cell group.

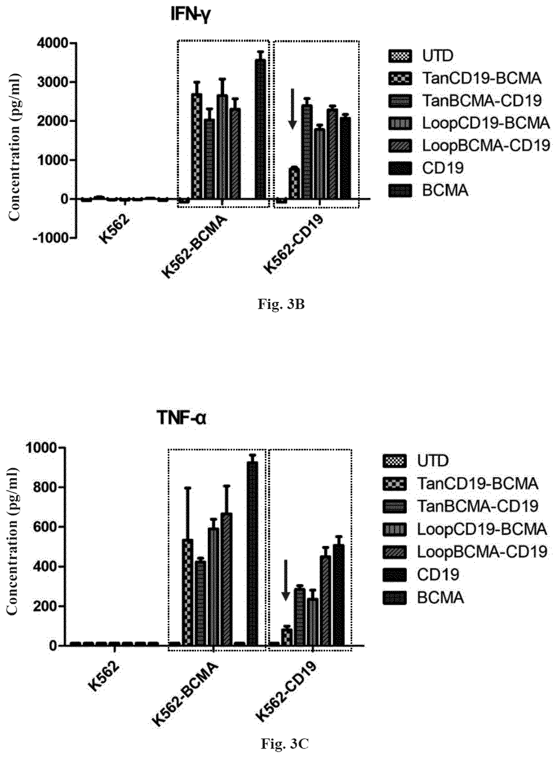

Figs. 3A-3C show cytokine release after various BCMA-CD19 bispecific CARs, CD19 CAR, and BCMA CAR activated by positive target cells in Example 4 of the present application; wherein Fig. 3A shows the release of IL-2 in each group, Fig. 3B shows the release of IFN-γ in each group, and Fig. 3C shows the release of TNF-α in each group. Among them, Figs. 3A, 3B, and 3C each contains two dotted boxes, and in the first dotted box in Figs. 3A, 3B, and 3C, five bars from left to right sequentially represent cytokine release of UTD cell, TanCD19-BCMA cell, TanBCMA-CD19 cell, LoopCD19-BCMA cell, LoopBCMA-CD19 cell, and BCMA cell after activated by K562-BCMA cell; in the second dotted box in Figs. 3A, 3B, and 3C, five bars from left to right sequentially represent cytokine release of UTD cell, TanCD19-BCMA cell, TanBCMA-CD19 cell, LoopCD19-BCMA cell, LoopBCMA-CD19 cell, and CD19 cell after activated by K562-CD19 cell.

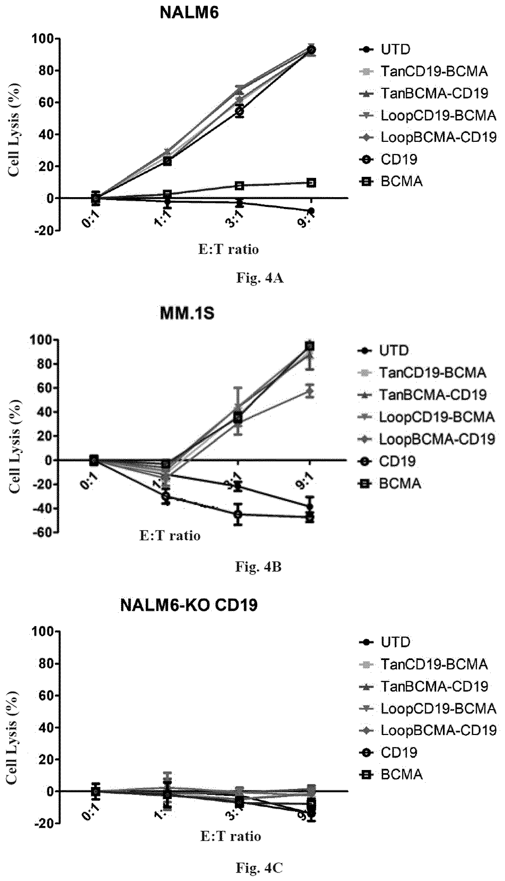

Figs. 4A-4C show the killing effects of various BCMA-CD19 bispecific CARs, CD19 CAR, and BCMA CAR cells on different target cells in Example 5 of the present application; wherein Fig. 4A is NALM6 group, which is a CD19 + BCMA-target cell; Fig. 4B is MM.1S group, which is BCMA+CD19-target cell; Fig. 4C is NALM6-KO CD19 group, which is a negative target cell control with CD19 knocked out while also not expressing BCMA.

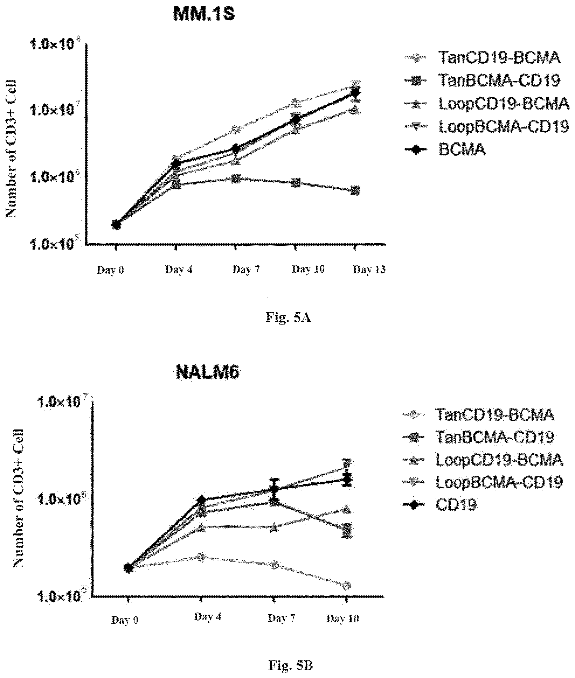

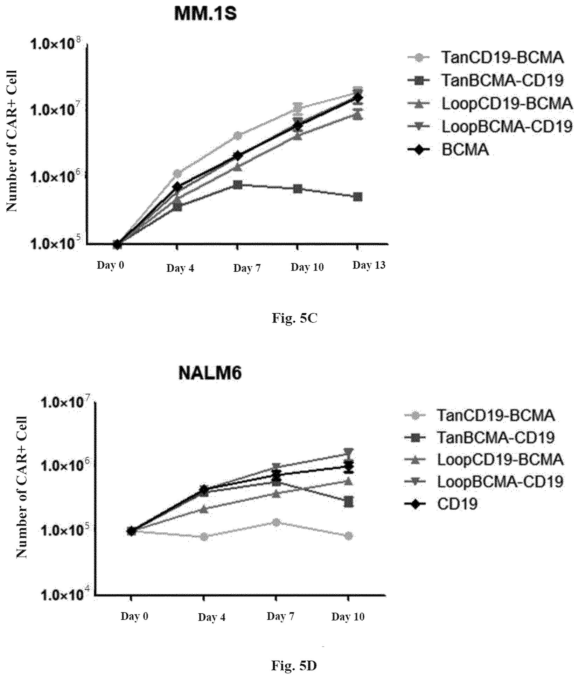

Figs. 5A-5D show the sustained proliferation of CAR-T cells in each group after multiple rounds of antigen stimulation in Example 6 of the present application; wherein Fig. 5A shows the proliferation of CD3 + cell stimulated with MM.1S cell, Fig. 5B shows the proliferation of CD3 + cell stimulated with NALM6 cell, Fig. 5C shows the proliferation of CAR + cell stimulated with MM.1S cell, and Fig. 5D shows the proliferation of CAR + cell stimulated with NALM6 cell.

### DETAILED DESCRIPTION OF THE INVENTION

The embodiments of the present invention will be illustrated with the following specific examples. Those familiar with this technology can easily understand other advantages and effects of the present invention from the disclosures in the present description.

The present application is further described below: in the present invention, unless otherwise stated, the scientific and technical terms used herein have the meanings commonly understood by those skilled in the art. Furthermore, the terms and laboratory procedures related to protein and nucleic acid chemistry, molecular biology, cell and tissue culture, microbiology, and immunology used herein are terms and routine procedures widely used in the corresponding fields. Meanwhile, in order to better understand the present invention, definitions and explanations of relevant terms are provided below.

In the present application, the term "Chimeric Antigen Receptor (CAR)" is the core component of CAR cell therapy, which may include an extracellular antigen recognition domain (for example, the portion that binds to Tumor-Associated Antigen (TAA), a hinge region, a transmembrane region and an intracellular domain. CAR-T (Chimeric Antigen Receptor T) cellular immunotherapy is considered to be one of the most promising methods to conquer tumors. CAR-T cells use genetic modification methods to make T cells express CAR proteins, and such CAR protein has the ability to recognize intact proteins on the membrane surface without depending on antigen presentation, thereby causing T cell activation and functional effects.

In the present application, the term "extracellular antigen recognition domain" refers to the Antigen Recognition Domain (ARD). The reason why CAR cell therapy products (such as CAR-T cells) can specifically recognize and/or bind to target antigens expressed by tumor cells depends on the extracellular antigen recognition domain. So far, the antigen recognition domain has been derived from Single Chain Variable Fragment (abbreviated as scFv) of an antibody, or from receptor-ligand interactions, TCR mimics, Variable Lymphocyte Receptors (VLR). So far, the most common source is scFv segment of the antibody. A scFv comprises antibody heavy chain variable regions (VH regions) and light chain variable regions (VL regions), which are connected by a peptide chain, such as the linker sequence GSTSGSGKPGSGEGSTKG consisting of 18 amino acids. The scFv antibody targeting two or more targets includes VH regions and VL regions targeting different targets, wherein the different regions are directly or indirectly linked via a linking sequence, and the arrangement thereof can be any one of the following forms: target 1 VL-target 1 VH-target 2 VL-target 2 VH, target 2 VL-target 2 VH-target 1 VL-target 1 VH, target 1 VL-target 2 VL-target 2 VH-target 1 VH, target 2 VL-target 1 VL-target 1 VH-target 2 VH, the above "-" represents linking via a linking sequence.

In the present application, the term "specific recognition and/or binding" refers to the recognition and/or binding between CAR and specific targets, CAR binding to this target with greater affinity, avidity, more easily, and/or with greater duration than CAR binding to other targets.

In the present application, the term "hinge region" refers to the linker segment between the extracellular antigen recognition domain and the transmembrane domain. This region allows the CAR to recognize the antigen by providing a certain range of activity to the antigen recognition domain. The hinge regions currently used are mainly derived from one or more of IgG1, IgG4, CD4, CD7, CD28, CD84, and CD8α. In addition, the typical hinge region also contains some residues that participate in CAR dimerization and contribute to enhance antigen sensitivity.

In the present application, "transmembrane region" refers to the transmembrane domain that connects the intracellular and extracellular components of the CAR structure. Different transmembrane domains can affect the expression and stability of CAR to some extent, but they are not directly involved in signal transduction, and they improve downstream signal transduction by interactions. The transmembrane region may be derived from one or more of CD3, CD4, CD7, CD8α, CD28, CD80, CD86, CD88, 4-1BB, CD152, OX40, and Fc70.

In present application, the term "intracellular domain" comprises an intracellular signal transduction region and may further comprise a costimulatory signal transduction region.

In the present application, the term "intracellular signal transduction region" refers to the activation of at least one normal effector function of immune effector cells responsible for expressing CAR. The intracellular signal transduction region may be derived from one or more of CD3ζ, CD3γ, CD3δ, CD3ε, CD5, CD22, CD79a, CD79b, FcRγ, FcRβ, CD66d, DAP10, DAP12, and Syk.

In the present application, the term "costimulatory signal transduction region" exists because in addition to stimulation by antigen-specific signal, many immune effector cells require costimulation to promote cell proliferation, differentiation, and survival, as well as effector function of activated cells. In some examples, the CAR may further comprise one or more costimulatory signal transduction region, wherein the costimulatory signal transduction region may be derived from one, two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3 and MyD88.

In the present application, the term "isolated" generally means those obtained from the natural state by artificial means. If an "isolated" substance or component occurs in nature, it may be that the natural environment in which it is located has changed, or that the substance has been separated from its natural environment, or both. For example, a certain unisolated polynucleotide or polypeptide naturally exists in a living animal, and the high purity of the same polynucleotide or polypeptide isolated from this natural state is called isolated. The term "isolated" does not exclude substances that have been artificially or synthetically obtained from their natural state by artificial means, nor does it exclude the presence of other impure substances that do not affect the activity of the substance.

In the present application, the term "guiding peptide" refers to the short peptide before the extracellular antigen recognition domain (such as the scFv sequence), whose function is to guide the recombinant protein synthesized in the cell to be exported to the outside of the cell. Commonly used guiding peptides include human CD8α signal peptide or human GM-CSF receptor α signal peptide.

In the present application, one of the key factors that determine the therapeutic effect of CAR-immune cells is the selection of tumor target antigens. In the present application, the term "BCMA" refers to B cell maturation antigen, and is a member of the tumor necrosis factor receptor superfamily. Human BCMA is expressed almost exclusively on plasma cells and multiple myeloma cells. BCMA may be a suitable tumor antigen target for immunotherapeutic agent against multiple myeloma. However, due to the heterogeneity of specific antigens on the surface of multiple myeloma cells, the selection of its antigen target is not necessarily a single one. By selecting appropriate targets, the anti-tumor activity of CAR-T cells can be optimized. The "CD19" molecule is currently the main target for treating blood system tumors derived from B lymphocytes, and is also a hot topic in CAR-T cell therapy research. Most malignant tumor cells derived from B cells express CD19 molecules on their surface. Multiple myeloma, as a B cell line tumor, generally does not express CD19 molecules. Therefore, CD19 is usually not used as a target for the treatment of multiple myeloma. However, some literature studies suggest that some trace amounts of drug-resistant and relapsed multiple myeloma clones also have the CD19⁺ phenotype. When using this dual-target CAR-T product, as long as one tumor antigen target is recognized, the CAR-T cells can be activated to prevent tumor antigen escape. Meanwhile, compared with preparing CAR-immune cells targeting different targets respectively and using them together, dual-target CAR immune cells have the following advantages: 1. it requires fewer immune cells, is easy to prepare and saves costs; 2. in terms of administration, the safety and operability of administrating one product are much higher than those of administrating two products.

In the present application, the term "linker sequence" generally refers to an oligopeptide or polypeptide region of about 1 to 100 amino acids in length, which connects any structure/region of the chimeric antigen receptor of the present invention together. The linker sequence may be consisted of different amino acid residues (e.g., glycine and serine) so that adjacent protein domains can move freely relative to each other. Longer linker sequences may be used when it is desirable to ensure that two adjacent domains do not spatially interfere with each other.

In the present application, the term "isolated nucleic acid molecule" generally refers to an isolated form of a nucleotide, deoxyribonucleotide or ribonucleotide of any length, which may be isolated from its natural environment or a synthetic analog.

In the present application, when performing CAR gene transduction/transfection and target gene expression, gene transduction/transfection methods mainly include viral and non-viral methods. For example: the methods through gamma retroviral vector, lentiviral vector, adenovirus-associated viral vector, plasmid DNA-dependent vector, transposon-dependent gene transfer, and mRNA-mediated gene transduction.

The term "vector" generally refers to a nucleic acid delivery vehicle into which a polynucleotide encoding a protein can be inserted and the protein can be expressed.

The vector can be transformed, transduced or transfected into the host cell so that the genetic material elements it carries can be expressed in the host cell. For example, vectors include: plasmids; phagemids; cosmids; artificial chromosomes such as yeast artificial chromosomes (YAC), bacterial artificial chromosomes (BAC) or P1-derived artificial chromosomes (PAC); phages such as lambda phage or M13 phage and animal viruses, etc. The types of animal viruses used as vectors include retroviruses (including lentiviruses), adenoviruses, adeno-associated viruses, herpesviruses (such as herpes simplex virus), poxviruses, baculoviruses, papillomaviruses, and papillomavirus (such as SV40). A vector may contain a variety of elements that control expression, including promoter sequences, transcription initiation sequences, enhancer sequences, selection elements, and reporter genes. In addition, the vector may also contain a replication origin. Vectors may also contain components that assist them in entering into cells, such as viral particles, liposomes, or protein coats, but not just these substances. The term "transposon" refers to discontinuous DNA segments that have the ability to migrate and carry genetic information between chromosomal sites, such as the Sleeping Beauty SB system and the PB system derived from lepidopteran insects. In some examples, mRNA can also be transduced into T cells by means of electrotransformation.

In the present application, the term "immune effector cell" generally refers to cell that participates in immune response, for example, the cell that promotes immune effector response. Immune effector cell can be selected from the following groups: one or more of T lymphocyte, natural killer cell (NK cell), peripheral blood mononuclear cell (PBMC cell), pluripotent stem cell, T lymphocyte differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK), and embryonic stem cell.

In the present application, the term "pharmaceutical composition" generally refers to a pharmaceutical composition suitable for administration to a patient, which may comprise the immune effector cell as described in the present application, and may further comprise one or more pharmaceutically acceptable adjuvant, such as: one or more of carrier, protective agent, stabilizer, excipient, diluent, solubilizer, surfactant, emulsifier and preservative. In some examples, pharmaceutically acceptable adjuvant comprises a protective agent, such as cell cryopreservation solution. In some examples, the pharmaceutical composition of the present application is a cell suspension or cryopreserved cell thereof.

In the present application, the term "subject" generally refers to a human or non-human animal, including but not limited to mouse, rat, cat, dog, rabbit, horse, pig, cow, sheep, or monkey.

In the present application, the term "comprise/comprising" generally refers to the inclusion of explicitly specified features, but not the exclusion of other elements.

In the present application, the term "about" generally refers to a range of fluctuations above or below the specified value that is acceptable to those skilled in the art, such as: changes within the range of ±0.5%-10%, such as changes within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below the specified value.

### Chimeric antigen receptor, nucleic acid, vector, immune effector cell, pharmaceutical composition

In one aspect, the present application provides a bispecific chimeric antigen receptor targeting BCMA-CD19, comprising an extracellular antigen recognition domain, a hinge region, a transmembrane region and an intracellular domain; wherein the extracellular antigen recognition domain comprises an anti-BCMA extracellular antigen recognition domain and an anti-CD19 extracellular antigen recognition domain;
the anti-BCMA extracellular antigen recognition domain comprises BCMA VH and BCMA VL, wherein amino acid sequences of BCMA VH complementarity-determining regions CDR1, CDR2 and CDR3 respectively comprise amino acid sequences as represented by SEQ ID NO: 1, SEQ ID NO: 2 and SEQ ID NO: 3, and amino acid sequences of BCMA VL complementarity-determining regions CDR1, CDR2 and CDR3 respectively comprise amino acid sequences as represented by SEQ ID NO: 4, SEQ ID NO: 5 and SEQ ID NO: 6.

In antibodies, the common rules for dividing CDR include Kabat, AbM, Chothia, Contact, and IMGT. These rules are well-known to those skilled in the art. When applying the website that implements these rules, once the VH and VL sequences are entered and the corresponding rules are selected, CDR sequences based on different rules can be obtained. In the present application, CDR is divided by using IMGT rule. However, those skilled in the art should understand that the protection scope of the present application covers combinations of CDR sequences obtained by analyzing using different rules.

In some examples, the anti-CD19 extracellular antigen recognition domain comprises CD19 VH and CD19 VL, wherein amino acid sequences of CD19 VH complementarity-determining regions CDR1, CDR2, and CDR3 respectively comprise amino acid sequences as represented by SEQ ID NO: 7, SEQ ID NO: 8 and SEQ ID NO: 9, and amino acid sequences of CD19 VL complementarity-determining regions CDR1, CDR2, and CDR3 respectively comprise amino acid sequences as represented by SEQ ID NO: 10, SEQ ID NO: 11 and SEQ ID NO: 12.

In some examples, the BCMA VH sequence comprises the amino acid sequence as represented by SEQ ID NO: 13, and the BCMA VL sequence comprises the amino acid sequence as represented by SEQ ID NO: 14.

In some examples, the BCMA VH sequence comprises the amino acid sequence as represented by SEQ ID NO: 15, and the BCMA VL sequence comprises the amino acid sequence as represented by SEQ ID NO: 16.

In some examples, the CD19 VH sequence comprises the amino acid sequence as represented by SEQ ID NO: 17, and the CD19 VL sequence comprises the amino acid sequence as represented by SEQ ID NO: 18.

In some examples, the present application also includes the substitution, deletion, addition and/or insertion of one or more amino acids in the amino acid sequence of any one of the above-mentioned bispecific chimeric antigen receptor; and their activity equivalent to any one of the above-mentioned chimeric antigen receptor. Those skilled in the art know that during the process of humanization, the amino acids in the FR regions in the VH and VL sequences can be substituted so that the CDR region of the modified antibody can still retain a suitable antigen-binding site. Therefore, the present application certainly includes different amino acid sequences obtained by humanizing the FR regions in the VH and VL sequences based on the above-mentioned CDR. Moreover, those skilled in the art also know that in the process of humanization, in order to ensure that the CDR region of the modified antibody can retain a suitable antigen-binding site, if necessary, 1, 2, 3, or no more than 10% of amino acid sequences in CDR may be substituted, deleted, added, and/or inserted, which are also included in the present application.

In some examples, the extracellular antigen recognition domain of the bispecific chimeric antigen receptor comprises any one structure selected from the group consisting of: CD19 VL sequence-1st linker sequence-CD19 VH sequence-2nd linker sequence-BCMA VL sequence-3rd linker sequence-BCMA VH sequence, BCMA VL sequence-4th linker sequence-BCMA VH sequence-5th linker sequence-CD19 VL sequence-6th linker sequence-CD19 VH sequence, BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence.

In some examples, the extracellular antigen recognition domain of the bispecific chimeric antigen receptor comprises any one structure selected from the group consisting of: BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence.

In some examples, the 1st linker sequence, the 2nd linker sequence, the 3rd linker sequence, the 4th linker sequence, the 5th linker sequence, the 6th linker sequence, the 7th linker sequence, the 8th linker sequence, the 9th linker sequence, the 10th linker sequence, the 11th linker sequence, and the 12th linker sequence are each independently selected from one or more of the following sequences: SEQ ID NO: 34, SEQ ID NO: 35, and SEQ ID NO: 36.

In some examples, the extracellular antigen recognition domain of the bispecific chimeric antigen receptor comprises the amino acid sequence as represented by SEQ ID NO: 19 or SEQ ID NO: 20.

In some examples, the hinge region is derived from one or more of IgG1, IgG4, CD4, CD7, CD28, CD84 and CD8α; optionally, the amino acid sequence of the hinge region is derived from CD8α; further optionally, the amino acid sequence of the hinge region comprises the amino acid sequence as represented by SEQ ID NO: 21.

In some examples, the transmembrane region is derived from one or more of CD3, CD4, CD7, CD8α, CD28, CD80, CD86, CD88, 4-1BB, CD152, OX40 and Fc70; optionally, the amino acid sequence of the transmembrane region is derived from CD8α; further optionally, the amino acid sequence of the transmembrane region comprises the amino acid sequence as represented by SEQ ID NO: 22.

In some examples, the intracellular domain comprises an intracellular signal transduction region; optionally, the intracellular domain further comprises a costimulatory signal transduction region; further optionally, the intracellular signal transduction region is derived from one or more of CD3ζ, CD3γ, CD3δ, CD3ε, CD5, CD22, CD79a, CD79b, FcRγ, FcRβ, CD66d, DAP10, DAP12 and Syk; even further optionally, the intracellular signal transduction region is derived from CD3ζ, for example, the amino acid sequence of the intracellular signal transduction region comprises the amino acid sequence as represented by SEQ ID NO: 23.

In some examples, the costimulatory signal transduction region is derived from one, two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3 and MyD88; optionally, the costimulatory signal transduction region is derived from CD28 or 4-1BB; further optionally, the amino acid sequence of the costimulatory signal transduction region comprises the amino acid sequence as represented by SEQ ID NO: 24.

In some examples, the bispecific chimeric antigen receptor further comprises a guiding peptide located at the N-terminus of the amino acid sequence of the chimeric antigen receptor; optionally, the guiding peptide is derived from CD8α; further optionally, the amino acid sequence of the guiding peptide comprises the amino acid sequence as represented by SEQ ID NO: 25.

In some examples, the bispecific chimeric antigen receptor comprises the amino acid sequence as represented by SEQ ID NO: 28 or SEQ ID NO: 29.

In another aspect, the present application also provides an isolated nucleic acid molecule comprising a nucleotide sequence encoding the above-mentioned bispecific chimeric antigen receptor.

In some examples, the nucleotide sequence encoding the bispecific chimeric antigen receptor comprises:
1) a nucleotide sequence encoding the BCMA VH amino acid sequence as represented by SEQ ID NO: 13, which is represented by SEQ ID NO: 37; and a nucleotide sequence encoding the BCMA VL amino acid sequence as represented by SEQ ID NO: 14, which is represented by SEQ ID NO: 38; and/or
2) a nucleotide sequence encoding the CD19 VH amino acid sequence as represented by SEQ ID NO: 17, which is represented by SEQ ID NO: 39; and a nucleotide sequence encoding the CD19 VL amino acid sequence as represented by SEQ ID NO: 18, which is represented by SEQ ID NO: 40.

In another aspect, the present application also provides a vector comprising the above-mentioned isolated nucleic acid molecule. Vectors include: plasmids; phagemids; cosmids; artificial chromosomes such as yeast artificial chromosomes (YAC), bacterial artificial chromosomes (BAC) or P1-derived artificial chromosomes (PAC); phages such as lambda phage or M13 phage and animal viruses, etc. The types of animal viruses used as vectors include retroviruses (including lentiviruses), adenoviruses, adeno-associated viruses, herpesviruses (such as herpes simplex virus), poxviruses, baculoviruses, papillomaviruses, and papillomavirus (such as SV40).

In some examples, the vector is an expression vector; optionally, the vector is a viral vector; further optionally, the vector is a lentiviral vector.

In another aspect, the present application also provides an engineered immune effector cell comprising the above-mentioned chimeric antigen receptor, the above-mentioned isolated nucleic acid molecule, or the above-mentioned vector.

In some examples, the engineered immune effector cell is selected from one or more of T lymphocyte, natural killer cell (NK cell), peripheral blood mononuclear cell (PBMC cell), pluripotent stem cell, T cell differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK) and embryonic stem cell.

In some examples, the engineered immune effector cell is T lymphocyte; optionally, the source of the T lymphocyte is autologous T lymphocyte or allogeneic T lymphocyte.

In some examples, the chimeric antigen receptor as described in the present application can be expressed or is expressed on the surface of the engineered immune effector cell.

In another aspect, the present application also provides a pharmaceutical composition comprising the above-mentioned engineered immune effector cell, and pharmaceutically acceptable adjuvant. The pharmaceutically acceptable adjuvant include one or more of carrier, protective agent, stabilizer, and diluent.

In some examples, the pharmaceutically acceptable adjuvant comprises a protective agent, such as cell cryopreservation solution.

In some examples, the pharmaceutical composition is a cell suspension or cryopreserved cell thereof.

In some examples, the pharmaceutical composition is an intravenous injection.

### Preparation method

In another aspect, the present application also provides a method for preparing engineered immune effector cell, including the following steps: transducing the vector as described in the present application into the immune effector cell.

In some examples, the engineered immune effector cell is selected from one or more of T lymphocyte, natural killer cell (NK cell), peripheral blood mononuclear cell (PBMC cell), pluripotent stem cell, T cell differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK) and embryonic stem cell.

In some examples, the engineered immune effector cell is T lymphocyte; optionally, the source of the T lymphocyte is autologous T lymphocyte or allogeneic T lymphocyte.

### Use

In another aspect, the present application also provides use of the bispecific chimeric antigen receptor, the isolated nucleic acid molecule, the vector and/or the engineered immune effector cell as described in the present application in the preparation of a medicament, wherein the medicament is used for the treatment of a disease or condition associated with the expression of BCMA.

In some examples, the disease or condition associated with the expression of BCMA is cancer; optionally, the cancer is multiple myeloma; further optionally, the cancer is refractory or relapsed multiple myeloma.

In some examples, the disease or condition associated with the expression of BCMA may be an autoimmune disease.

In some examples, the autoimmune disease may be selected from the group consisting of systemic lupus erythematosus, rheumatoid arthritis, idiopathic thrombocytopenic purpura, myasthenia gravis, and autoimmune hemolytic anemia.

In another aspect, the present application also provides a method for treating a disease or condition associated with the expression of BCMA, including the following steps: administering an effective dose of the chimeric antigen receptor, the isolated nucleic acid molecule, the vector, and/or the engineered immune effector cell as described the present application to a subject having a need to treat a disease or condition associated with the expression of BCMA.

In some examples, the disease or condition associated with the expression of BCMA is cancer; optionally, the cancer is multiple myeloma; further optionally, the cancer is refractory or relapsed multiple myeloma.

In some examples, the disease or condition associated with the expression of BCMA may be an autoimmune disease.

In some examples, the autoimmune disease may be selected from the group consisting of systemic lupus erythematosus, rheumatoid arthritis, idiopathic thrombocytopenic purpura, myasthenia gravis, and autoimmune hemolytic anemia.

In some examples, the administration can be carried out in different ways, such as intravenous, intratumoral, intraperitoneal, subcutaneous, intramuscular, topical or intradermal administration. For example, the method of administration may be administered to a subject by intravenous injection. In some examples, an effective dose of engineered immune effector cell or the pharmaceutical composition can be administered to a subject in a single time, or in divided doses within a certain period, such as once a week, once every two weeks, once every three weeks, once every four weeks, once a month, once every 3 months, or once every 3-6 months.

In some examples, the doses for administration may vary for different indications; and the doses for administration may also vary depending on the severity of the patient's condition. The dose may be administered in a range of 1×10⁵ CAR positive T cells/kg to 1×10⁷ CAR positive T cells/kg, for example, 1×10⁵ CAR positive T cells/kg to 1×10⁶ CAR positive T cells/kg, 1×10⁶ CAR positive T cells/kg to 1×10⁷ CAR positive T cells/kg, 0.5×10⁶ CAR positive T cells/kg, 0.6×10⁶ CAR positive T cells/kg, 0.7×10⁶ CAR positive T cells/kg, 0.8×10⁶ CAR positive T cells/kg, 0.9×10⁶ CAR positive T cells/kg, 1.0×10⁶ CAR positive T cells/kg, 1.1×10⁶ CAR positive T cells/kg, 1.2×10⁶ CAR positive T cells/kg, 1.3×10⁶ CAR positive T cells/kg, 1.4×10⁶ CAR positive T cells/kg, 1.5×10⁶ CAR positive T cells/kg, 1.6×10⁶ CAR positive T cells/kg, 1.7×10⁶ CAR positive T cells/kg, 1.8×10⁶ CAR positive T cells/kg, 1.9×10⁶ CAR positive T cells/kg, 2.0×10⁶ CAR positive T cells/kg.

In some examples, the subject may include human and non-human animal. For example, the subject may include, but not limited to, mouse, rat, cat, dog, horse, pig, cow, sheep, rabbit, or monkey.

In another aspect, the present application also provides the chimeric antigen receptor, the isolated nucleic acid molecule, the vector and/or the engineered immune effector cell, for use in treating a disease or condition associated with the expression of BCMA.

In some examples, the disease or condition associated with expression of BCMA may include non-solid tumor; optionally, the non-solid tumor is a hematological tumor.

In some examples, the disease or condition associated with expression of BCMA may include multiple myeloma.

In some examples, the multiple myeloma is relapsed or refractory multiple myeloma.

Without wishing to be bound by any theory, the following examples are only described to illustrate the chimeric antigen receptor, the engineered immune effector cell, the preparation method and use of the present application, and are not used to limit the scope thereof. The examples do not include detailed descriptions of traditional methods, such as those used to construct vectors and plasmids, methods of inserting genes encoding proteins into such vectors and plasmids, or methods of introducing plasmids into host cells. Such methods have been well-known to those of ordinary skill in the art, and described in many publications, including Sambrook, J., Fritsch, E. F. and Maniais, T. (1989) Molecular Cloning: A Laboratory Manual, 2nd edition, Cold spring Harbor Laboratory Press.

### Examples

### Example 1. Obtaining BCMA-CD19 bispecific CAR-T cells

We have obtained a BCMA-specific humanized antibody (the amino acid sequence of its VH is represented by SEQ ID NO: 13, the nucleotide sequence of its VH is represented by SEQ ID NO: 37, the amino acid sequence of its VL is represented by SEQ ID NO: 14, the nucleotide sequence of its VL is represented by SEQ ID NO: 38, amino acid sequences of its VH CDR1, CDR2, and CDR3 are represented by SEQ ID NO: 1, SEQ ID NO: 2, and SEQ ID NO: 3, respectively, and amino acid sequences of its VL CDR1, CDR2, and CDR3 are represented by SEQ ID NO: 4, SEQ ID NO: 5, and SEQ ID NO: 6, respectively) and a CD19-specific antibody (the amino acid sequence of its VH is represented by SEQ ID NO: 17, the nucleotide sequence of its VH is represented by SEQ ID NO: 39, the amino acid sequence of its VL is represented by SEQ ID NO: 18, the nucleotide sequence of its VL is represented by SEQ ID NO: 40, the amino acid sequences of its VH CDR1, CDR2, and CDR3 are represented by SEQ ID NO: 7, SEQ ID NO: 8, SEQ ID NO: 9, respectively, and the amino acid sequences of its VL CDR1, CDR2, and CDR3 are represented by SEQ ID NO: 10, SEQ ID NO: 11, and SEQ ID NO: 12, respectively), in the hope of obtaining a superior bispecific CAR structure. Considering the changes of the order of the targets BCMA and CD19, and the order of VH and VL in the extracellular antigen recognition domain of each target, etc., there are multiple options for designing the structure of BCMA-CD19 bispecific CAR. We chose to screen candidate BCMA-CD19 bispecific CAR structures on the second-generation CAR structures.

We tried six BCMA-CD19 bispecific CAR structures as represented by Fig. 1, and used BCMA CAR structure and CD19 CAR structure as controls. In each CAR structure, the following structures were used in this example: CD8α guiding chain as the signal peptide (as represented by SEQ ID NO: 25), the structure of CD8α as the hinge region (as represented by SEQ ID NO: 21) and transmembrane region (as represented by SEQ ID NO: 22), 4-1BB as the intracellular costimulatory signal (as represented by SEQ ID NO: 24), and CD3ζ as the T cell activation signal (as represented by SEQ ID NO: 23).

### 1. Construction of lentiviral vectors

The six BCMA-CD19 bispecific CAR structures, as well as the BCMA CAR structure and the CD19 CAR structure as controls as represented by Fig. 1 were artificially synthesized, and amino acid sequences of Tan CD19-BCMA structure, Tan BCMA-CD19 structure, Loop CD19-BCMA structure, Loop BCMA-CD19 structure, CD19-2A-BCMA structure, BCMA-2A-CD19 structure, CD19 CAR structure, and BCMA CAR structure are represented by SEQ ID NO: 26, SEQ ID NO: 27, SEQ ID NO: 28, SEQ ID NO: 29, SEQ ID NO: 30, SEQ ID NO: 31, SEQ ID NO: 32, and SEQ ID NO: 33, respectively. The description as below only briefly summarized the main distinguishing parts in the CAR structures, and did not fully display the signal peptide, linker sequence, hinge region, transmembrane region, intracellular costimulatory signal region, and T cell activation signal region. The order of each structure can be seen from Fig. 1: SP represented the signal peptide region, Hinge represented the hinge region, TM represented the transmembrane region, and the linker sequence was between VL and VH.
1) Tan CD19-BCMA structure: CD19 VL-CD19 VH-BCMA VL-BCMA VH;
2) Tan BCMA-CD19 structure: BCMA VL-BCMA VH-CD19 VL-CD19 VH;
3) Loop CD19-BCMA structure: BCMA VL-CD19 VL-CD19 VH-BCMA VH;
4) Loop BCMA-CD19 structure: CD19 VL-BCMA VL-BCMA VH-CD19 VH;
5) CD19-2A-BCMA structure: CD19 CAR-T2A-BCMA CAR (there was a complete CD19 CAR structure and BCMA CAR structure in front of and behind the self-cleaving polypeptide T2A of this structure, respectively. The complete CAR structure referred to the same signal peptide, hinge region, transmembrane region, intracellular costimulatory signal region, and T cell activation signal region as those in other groups);
6) BCMA-2A-CD19 structure: BCMA CAR-T2A-CD19 CAR (there was a complete BCMA CAR structure and CD19 CAR structure in front of and behind the self-cleaving polypeptide T2A, respectively. The complete CAR structure referred to the same signal peptide, hinge region, transmembrane region, intracellular costimulatory signal region, and T cell activation signal region as those in other groups);
7) CD19 CAR structure: CD19 VL-CD19 VH;
8) BCMA CAR structure: BCMA VL-CD19 VH.

The above-mentioned six BCMA-CD19 bispecific CAR structures, one CD19 CAR structure, and one BCMA CAR structure were respectively constructed into modified empty lentiviral vectors (manufacturer: SBI Corporation, catalog number: CD500-CD800, conventional resistance modification was performed as described in Example 1 of WO2021/121227) to obtain CAR expression vectors, and then the CAR expression vectors and three packaging plasmids were transfected into 293T cells together, and functional lentiviral vectors were obtained after collection and purification. The three packaging plasmids were PMD2.0G (purchased from Biovector Corporation, catalog number: Biovector 012259), pMDLg/pRRE (purchased from Biovector Corporation, catalog number: Biovector 012251), and pRSV-Rev (purchased from Biovector Corporation, catalog number: Biovector 012253), respectively.

2. Preparation of the corresponding six BCMA-CD19 bispecific CAR-T cells, one CD19 CAR-T cell, and one BCMA CAR-T cell by lentiviral transduction.

The transduction experiment was carried out according to conventional methods known to those skilled in the art. The steps of transduction were briefly described as follows:
1) Sorting of T cells
   Peripheral blood mononuclear cells (PBMC) were isolated from the subject's apheresis cells, and then T cells were sorted from the PBMC cells.
2) Activation of the T cells
   The isolated T cells were resuspended with complete lymphocyte culture medium (X-VIVO15 medium + 5% FBS + 300 IU/ml IL-2 or X-VIVO15 medium + 5% FBS + 5 ng/ml IL-15 + 10 ng/ml IL-7) to give a final concentration of (1~2)×10⁶ cells/ml, and 5 to 10 µl of CD3/CD28 stimulation magnetic beads were added. The mixture was well mixed, and placed in an incubator for culture for at least 24 h under the culture condition of 37°C+5% CO₂.
3) Transduction of T cells with lentiviruses
   The activated cultured T cells were taken out, and polybrene at a final concentration of 8 µg/ml was added and mixed well. The lentiviral vector was slowly added at MOI=2. After well mixed, the mixture was placed in a centrifuge and centrifuged at 1500 rpm for 1.5 h. After that, it was placed in an incubator for culture for at least 24 h under the culture condition of 37°C +5% CO₂.
4) Expansion culture of transduced T cells

The transduced cells were taken out and the cell density was monitored to keep it at (0.5~1)×10⁶ cells/ml for use in subsequent examples.

After T cells were infected with lentivirus containing Tan CD19-BCMA structure, Tan BCMA-CD19 structure, Loop CD19-BCMA structure, Loop BCMA-CD19 structure, CD19-2A-BCMA structure, BCMA-2A-CD19 structure, CD19 CAR, and BCMA CAR, the obtained T cells were named Tan CD19-BCMA cells, Tan BCMA-CD19 cells, Loop CD19-BCMA cells, Loop BCMA-CD19 cells, CD19-2A-BCMA cells, BCMA-2A-CD19 cells, CD19 cells, and BCMA cells, respectively. Next, we screened the six bispecific CAR structures at the cellular level to determine the characteristics of each CAR structure and select a superior bispecific CAR structure.

### Example 2. Detection of CAR molecules expressed on the surface of BCMA-CD19 bispecific CAR-T cells

The CAR protein molecules expressed on the surface of the six bispecific CAR-T cells obtained in Example 1 were detected. We used PE fluorescent-labeled CD19 antigen (manufacturer: ACRO Biosystems, catalog number: CD9-HP2H3) and FITC fluorescent-labeled BCMA antigen (manufacturer: ACRO Biosystems, catalog number: BCA-HF254) to stain the six BCMA-CD19 bispecific CAR-T cells and UTD cells (T cells without CAR transduction) obtained in Example 1, and performed the detection and analysis of CAR molecule positive proportion through flow cytometry. The inventors first discovered that: as represented by Fig. 2A, for CD19-2A-BCMA cells and BCMA-2A-CD19 cells, 6 days after T cell infection, the CAR molecules connected behind T2A were poorly expressed, so the two CAR structures in CD19-2A-BCMA cells and BCMA-2A-CD19 cells were eliminated first. As represented by Figs. 2B, 2C, 2D, and 2E: 6 days after T cell infection, the CAR expression rates of the other four bispecific CAR-T cells Tan CD19-BCMA cells, Tan BCMA-CD19 cells, Loop CD19-BCMA cells, and Loop BCMA-CD19 cells were 42.98%, 66.71%, 53.46%, and 61.63%, respectively (based on the results after BCMA antigen staining, the reason was recited in detail in Example 3).

### Example 3. Detection of CAR molecules on the surface of BCMA-CD19 bispecific CAR-T cells

The inventors have tried to perform single staining (single staining meant that the bispecific CAR-T cells were stained with only FITC fluorescent-labeled BCMA antigen or PE fluorescent-labeled CD19 antigen) and co-staining (co-staining meant that the bispecific CAR-T cells were stained with both FITC fluorescent-labeled BCMA antigen and PE fluorescent-labeled CD19 antigen) of the bispecific CAR-T cells after 11 days after T cell infection obtained in Example 1 to select the staining method. Because the extracellular moiety of BCMA and the extracellular moiety of CD19 were constructed on one scFv and were always co-expressed, in theory, the difference of results should be controlled within the error range whether bispecific CAR-T cells were stained through single staining method or co-staining method. However, we found that the selection of different antigens for staining had a significant impact on the calculation of CAR+ cells of bispecific CAR-T cells, as represented by Table 1 (especially the shaded part):

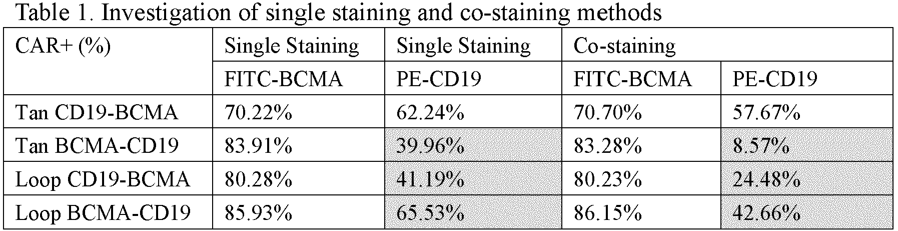

Taking the characterization of the proportion of CAR+ cells in Tan BCMA-CD19 cells as an example, when BCMA antigen was used for single staining and when BCMA antigen + CD19 antigen was used for co-staining, the proportions of CAR+ detected through BCMA antigen were all within the error range. However, when CD19 antigen was used for single staining and BCMA antigen + CD19 antigen were used for co-staining, the values detected through CD19 antigen fluctuated greatly, far exceeding the error range; and the proportions of CAR+ cells detected through CD19 antigen were significantly lower than those detected through BCMA antigen. Meanwhile, when CD19 antigen was used for detection, there was also a significant difference between the proportions of CAR+ cells detected through single staining and co-staining. Meanwhile, the above observations were not limited by the relative positions of the BCMA extracellular antigen recognition domain and the CD19 extracellular antigen recognition domain in the CAR structure. We suppose that compared with the CD19 antigen, the BCMA antigen is smaller and can more easily overcome the problems of the steric hindrance in the bispecific CAR structure to bind to the CAR protein. Therefore, in the subsequent experiments of the present application, the data of detection through BCMA antigen were used to characterize the positive proportion of BCMA-CD19 bispecific CAR; of course, the data of detection through BCMA antigen in co-staining method could also characterize the positive proportion of BCMA-CD19 bispecific CAR, but the single staining method was simpler.

### Example 4: Cytokine release experiment on BCMA-CD19 bispecific CAR-T cells

Cytokine release experiment: the four bispecific CAR-T cells (Tan CD19-BCMA, Tan BCMA-CD19, Loop CD19-BCMA, Loop BCMA-CD19), BCMA CAR-T cells, CD19 CAR-T cells and UTD cells obtained in Example 1 were co-cultured with target cells in X-VIVO15 medium at an effector-target ratio of 1:1 for 24 hours, and then the concentrations of IL-2, IFN-γ and TNF-α in the cell supernatant were detected through ELISA method; wherein K562 was a double-negative target cell of BCMA and CD19, K562-BCMA was a positive target cell that exogenously expressed BCMA but did not express CD19, and K562-CD19 was a positive target cell that exogenously expressed CD19 but did not express BCMA. Since K562-BCMA cells did not express CD19 antigen, they were not used to detect CD19 CAR-T cells. Similarly, since K562-CD19 cells did not express BCMA antigen, they were not used to detect BCMA CAR-T cells (in Fig. 3A, Fig. 3B, and Fig. 3C, the K562-BCMA group and the K562-CD19 group each lacked one bar).

The experimental results of the release of cytokines IL-2, IFN-γ, and TNF-α were represented by Figs. 3A, 3B, and 3C, respectively. The arrows in Figs. 3A, 3B, and 3C pointed to CAR-T cells with lower cytokine release levels. It can be seen from the figures that compared with the Tan CD19-BCMA structure and the Tan BCMA-CD19 structure, the levels of various cytokines released by the bispecific CARs of the Loop CD19-BCMA structure and the Loop BCMA-CD19 structure were higher overall. In particular, it can be seen from the cytokine release levels in the Tan CD19-BCMA structure and the Tan BCMA-CD19 structure that, when the scFv far away from the cell membrane in the Tan CD19-BCMA structure and the Tan BCMA-CD19 structure was stimulated by antigen, the level of cytokine released by CAR-T cells was relatively low.

### Example 5. Cell killing experiment on BCMA-CD19 bispecific CAR-T cells

Cell killing experiment: the four bispecific CAR-T cells (Tan CD19-BCMA, Tan BCMA-CD19, Loop CD19-BCMA, Loop BCMA-CD19), BCMA CAR-T cells, CD19 CAR-T cells and UTD cells obtained in Example 1 were co-cultured respectively with target cells in X-VIVO15 medium at different effector-target ratios (0:1, 1:1, 3:1 or 9:1) for 4 hours. Then the target cell killing ratio was detected by detecting the activity of luciferase stably expressed in the target cells. NALM6 (human acute lymphoblastic leukemia cells) were CD19+BCMA⁻ target cells, MM.1S (human multiple myeloma cells) were BCMA+CD19⁻ target cells, and NALM6⁻ KO CD19 was a negative target cell control in which CD19 was knocked out and BCMA was not expressed. The cell killing results were represented by Figs. 4A to 4C. In Fig. 4A, Tan CD19-BCMA cells, Tan BCMA-CD19 cells, Loop CD19-BCMA cells, Loop BCMA-CD19 cells, and CD19 CAR-T cells all had good killing effects on the positive target cells NALM6 that endogenously expressed CD19, while BCMA CAR-T cells and UTD cells had no killing effects on the positive target cells NALM6 that endogenously expressed CD19; in Fig. 4B, Tan CD19-BCMA cells, Tan BCMA-CD19 cells, Loop CD19-BCMA cells, Loop BCMA-CD19 cells, and BCMA CAR-T cells all had good killing effects on the positive target cells MM.1S that endogenously expressed BCMA, while CD19 CAR-T cells and UTD cells had no killing effect on the positive target cells MM.1S that endogenously expressed BCMA; in Fig. 4C, all cells had no killing effect on the negative target cell control in which CD19 was knocked out and BCMA was not expressed.

### Example 6. Sustained proliferation of BCMA-CD19 bispecific CAR-T cells

Antigen stimulation can activate CAR-T cells and cause CAR-T cell proliferation, while sustained activation of T cells will lead to cell exhaustion. The proliferation ability and effector function of exhausted T cells will decrease. We verified the sustained proliferation of BCMA-CD19 bispecific CAR-T cells by detecting the proliferation of CD3+ cells (i.e., T cell proliferation), the proliferation of CAR+ cells and the proportion of CAR+ cells after multiple rounds of antigen stimulation experiments.

Before antigen stimulation, the CAR positive proportions of the four bispecific CAR-T cells (Tan CD19-BCMA, Tan BCMA-CD19, Loop CD19-BCMA, Loop BCMA-CD19), BCMA CAR-T cells, and CD19 CAR-T cells obtained in Example 1 were all adjusted using UTD to a level consistent with that of the group of CAR-T cells with the lowest CAR positive proportion, wherein the CAR positive proportions of Tan CD19-BCMA cells, Tan BCMA-CD19 cells, Loop CD19-BCMA cells, Loop BCMA-CD19 cells, and BCMA CAR-T cells were based on the detection data of BCMA antigen, and the CAR positive proportion of CD19 CAR-T cells was based on the detection data of CD19 antigen. In multiple rounds of antigen stimulation experiments, each group of CAR-T cells was respectively co-cultured with positive target cells in a 24-well plate at an effector-target ratio of 1:2, with 2 ml of X-VIVO15 medium per well, and 3 wells were repeated for each group of cells. The positive target cells used were MM.1S and NALM6 respectively, which was equivalent to BCMA and CD19 as antigens respectively for multiple rounds of stimulation to test the effects of multiple rounds of stimulation with different antigens on the sustained proliferation of BCMA-CD19 bispecific CAR-T cells. Since MM.1S cells did not express CD19 antigen, they were not used to detect CD19 CAR-T cells. Similarly, since NALM6 cells did not express BCMA antigen, they were not used to detect BCMA CAR-T cells.

After CAR-T cells were co-cultured with positive target cells for 3 days, 500 µl of cells were taken out to perform CD3 and CAR staining with fluorescent-labeled CD3 antibody (manufacturer: BioLegend, catalog number: 300312) and BCMA antigen and CD19 antigen (same as in Example 2) (Tan CD19-BCMA cells, Tan BCMA-CD19 cells, Loop CD19-BCMA cells, Loop BCMA-CD19 cells, BCMA CAR-T cells were stained with BCMA antigen, and CD19 CAR-T cells were stained with CD19 antigen). Detection and analysis were performed by flow cytometry to display the proportion and number of CAR positive cells in CD3 positive cells. The number of CAR positive cells in CD3-positive cells can also be calculated based on the conversion of volume multiples (CD3 is a marker that distinguishes whether it is a T cell). Then, according to the calculation results, a certain amount of CAR-T cells were taken out from each group and added to the corresponding positive target cells at an effector-target ratio of 1:2 for a new round of stimulation, and this was repeated for 3-4 rounds of stimulation.

After multiple rounds of antigen stimulation experiments, the results of the proliferation of CD3+ cells stimulated with MM.1S and NALM6 cells respectively (i.e., the proliferation of T cells) were represented by Figs. 5A and 5B; the results of the proliferation of CAR+ cells stimulated with MM.1S and NALM6 cells respectively are represented by Figs. 5C and 5D. As represented by Figs. 5A and 5C, after multiple rounds of antigen stimulation experiments, the number of CD3+ cells (i.e., the proliferation of T cells) and the number of CAR+ cells in the Tan BCMA-CD19 cells stimulated with MM.1S cells were significantly lower than those in other groups of CAR-T cells; as represented by Figs. 5B and 5D, after multiple rounds of antigen stimulation experiments, the number of CD3+ cells and the number of CAR+ cells in the Tan CD19-BCMA cells stimulated with NALM6 cells were significantly lower than those in other groups of CAR-T cells; by comparing Fig. 5A with Fig. 5B, and comparing Fig. 5C with Fig. 5D, it can be found that the proliferation ability of CAR-T cells stimulated with MM.1S cells was stronger than that of CAR-T cells stimulated with NALM6 cells. The above results showed that when the scFv far away from the cell membrane in the Tan-structured BCMA-CD19 bispecific CAR-T cells was stimulated with antigens, the sustained proliferation ability of the CAR-T cells was relatively poor; while the sustained proliferation ability of the Loop-structured CAR-T cells was overall stronger than that of the Tan-structured ones, and the cells had the ability of sustained proliferation when stimulated with the two antigens of BCMA and CD19, wherein the proliferation ability of CAR-T cells stimulated with BCMA antigen was stronger.

In summary, *in vitro* pharmacodynamic tests showed that Tan CD19-BCMA, Tan BCMA-CD19, Loop CD19-BCMA, and Loop BCMA-CD19 structures had superior performances in terms of the expression on the surface of T cells, cytokine release, and *in vitro* cell killing; in terms of sustained proliferation of CAR-T, Loop CD19-BCMA and Loop BCMA-CD19 structures were significantly superior to Tan CD19-BCMA and Tan BCMA-CD19 structures and performed better. Moreover, the Loop CD19-BCMA and Loop BCMA-CD19 structures performed similarly to BCMA CAR-T and CD19 CAR-T in various experiments and tests, and had complete functionality for dual targets.

### The Sequence Description

SEQ ID NO: 1: BCMA VH CDR1;
SEQ ID NO: 2: BCMA VH CDR2;
SEQ ID NO: 3: BCMA VH CDR3;
SEQ ID NO: 4: BCMA VL CDR1;
SEQ ID NO: 5: BCMA VL CDR2 (ETS, Glu Thr Ser);
SEQ ID NO: 6: BCMA VL CDR3;
SEQ ID NO: 7: CD19 VH CDR1;
SEQ ID NO: 8: CD19 VH CDR2;
SEQ ID NO: 9: CD19 VH CDR3;
SEQ ID NO: 10: CD19 VL CDR1;
SEQ ID NO: 11: CD19 VL CDR2 (SAT, Ser Ala Thr);
SEQ ID NO: 12: CD19 VL CDR3;
SEQ ID NO: 13: BCMA VH sequence (Humanized);
SEQ ID NO: 14: BCMA VL sequence (Humanized);
SEQ ID NO: 15: BCMA VH sequence (Rabbit-derived);
SEQ ID NO: 16: BCMA VL sequence (Rabbit-derived);
SEQ ID NO: 17: CD19 VH sequence;
SEQ ID NO: 18: CD19 VL sequence;
SEQ ID NO: 19: Amino acid sequence of scFv in Loop CD19-BCMA structure;
SEQ ID NO: 20: Amino acid sequence of scFv in Loop BCMA-CD19 structure;
SEQ ID NO: 21: Amino acid sequence of hinge region;
SEQ ID NO: 22: Amino acid sequence of transmembrane region;
SEQ ID NO: 23: Amino acid sequence of the intracellular signal transduction region;
SEQ ID NO: 24: Amino acid sequence of costimulatory signal transduction region;
SEQ ID NO: 25: Amino acid sequence of the guiding peptide;
SEQ ID NO: 26: Amino acid sequence of Tan CD19-BCMA structure;
SEQ ID NO: 27: Amino acid sequence of Tan BCMA-CD19 structure;
SEQ ID NO: 28: Amino acid sequence of Loop CD19-BCMA structure;
SEQ ID NO: 29: Amino acid sequence of Loop BCMA-CD19 structure;
SEQ ID NO: 30: Amino acid sequence of CD19-2A-BCMA structure;
SEQ ID NO: 31: Amino acid sequence of BCMA-2A-CD19 structure;
SEQ ID NO: 32: Amino acid sequence of CD19 CAR structure;
SEQ ID NO: 33: Amino acid sequence of BCMA CAR structure;
SEQ ID NO: 34: Linker sequence;
SEQ ID NO: 35: Linker sequence;
SEQ ID NO: 36: Linker sequence;
SEQ ID NO: 37: Nucleotide sequence encoding the BCMA VH amino acid sequence as represented by SEQ ID NO: 13;
SEQ ID NO: 38: Nucleotide sequence encoding the BCMA VL amino acid sequence as represented by SEQ ID NO: 14;
SEQ ID NO: 39: Nucleotide sequence encoding the CD19 VH amino acid sequence as represented by SEQ ID NO: 17;
SEQ ID NO: 40: Nucleotide sequence encoding the CD19 VL amino acid sequence as represented by SEQ ID NO: 18.

## Claims

1. A bispecific chimeric antigen receptor targeting BCMA-CD19, comprising an extracellular antigen recognition domain, a hinge region, a transmembrane region and an intracellular domain; wherein the extracellular antigen recognition domain comprises an anti-BCMA extracellular antigen recognition domain and an anti-CD19 extracellular antigen recognition domain;
the anti-BCMA extracellular antigen recognition domain comprises BCMA VH and BCMA VL, wherein amino acid sequences of BCMA VH complementarity-determining regions CDR1, CDR2 and CDR3 respectively comprise amino acid sequences as represented by SEQ ID NO: 1, SEQ ID NO: 2 and SEQ ID NO: 3, and amino acid sequences of BCMA VL complementarity-determining regions CDR1, CDR2 and CDR3 respectively comprise amino acid sequences as represented by SEQ ID NO: 4, SEQ ID NO: 5 and SEQ ID NO: 6.

2. The bispecific chimeric antigen receptor according to claim 1, wherein the anti-CD19 extracellular antigen recognition domain comprises CD19 VH and CD19 VL, wherein amino acid sequences of CD19 VH complementarity-determining regions CDR1, CDR2 and CDR3 respectively comprise amino acid sequences as represented by SEQ ID NO: 7, SEQ ID NO: 8 and SEQ ID NO: 9, and amino acid sequences of CD19 VL complementarity-determining regions CDR1, CDR2 and CDR3 respectively comprise amino acid sequences as represented by SEQ ID NO: 10, SEQ ID NO: 11 and SEQ ID NO: 12.

3. The bispecific chimeric antigen receptor according to claim 1 or 2, wherein the BCMA VH sequence comprises the amino acid sequence as represented by SEQ ID NO: 13, and the BCMA VL sequence comprises the amino acid sequence as represented by SEQ ID NO: 14; or
the BCMA VH sequence comprises the amino acid sequence as represented by SEQ ID NO: 15, and the BCMA VL sequence comprises the amino acid sequence as represented by SEQ ID NO: 16.

4. The bispecific chimeric antigen receptor according to claim 2, wherein the CD19 VH sequence comprises the amino acid sequence as represented by SEQ ID NO: 17, and the CD19 VL sequence comprises the amino acid sequence as represented by SEQ ID NO: 18.

5. The bispecific chimeric antigen receptor according to claim 1, wherein the extracellular antigen recognition domain of the bispecific chimeric antigen receptor comprises any one structure selected from the group consisting of: CD19 VL sequence-1st linker sequence-CD19 VH sequence-2nd linker sequence-BCMA VL sequence-3rd linker sequence-BCMA VH sequence, BCMA VL sequence-4th linker sequence-BCMA VH sequence-5th linker sequence-CD19 VL sequence-6th linker sequence-CD19 VH sequence, BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence;
optionally, the extracellular antigen recognition domain of the bispecific chimeric antigen receptor comprises any one structure selected from the group consisting of: BCMA VL sequence-7th linker sequence-CD19 VL sequence-8th linker sequence-CD19 VH sequence-9th linker sequence-BCMA VH sequence, and CD19 VL sequence-10th linker sequence-BCMA VL sequence-11th linker sequence-BCMA VH sequence-12th linker sequence-CD19 VH sequence;
further optionally, the 1st linker sequence, the 2nd linker sequence, the 3rd linker sequence, the 4th linker sequence, the 5th linker sequence, the 6th linker sequence, the 7th linker sequence, the 8th linker sequence, the 9th linker sequence, the 10th linker sequence, the 11th linker sequence, and the 12th linker sequence are each independently selected from one or more of the following sequences: SEQ ID NO: 34, SEQ ID NO: 35, and SEQ ID NO: 36.

6. The bispecific chimeric antigen receptor according to claim 5, wherein the extracellular antigen recognition domain of the bispecific chimeric antigen receptor comprises the amino acid sequence as represented by SEQ ID NO: 19 or SEQ ID NO: 20.

7. The bispecific chimeric antigen receptor according to claim 1, wherein the hinge region is derived from one or more of IgG1, IgG4, CD4, CD7, CD28, CD84 and CD8α; optionally, the amino acid of the hinge region is derived from CD8α; further optionally, the amino acid sequence of the hinge region comprises the amino acid sequence as represented by SEQ ID NO: 21; and/or
wherein the transmembrane region is derived from one or more of CD3, CD4, CD7, CD8α, CD28, CD80, CD86, CD88, 4-1BB, CD152, OX40 and Fc70; optionally, the amino acid of the transmembrane region is derived from CD8α; further optionally, the amino acid sequence of the transmembrane region comprises the amino acid sequence as represented by SEQ ID NO: 22.

8. The bispecific chimeric antigen receptor according to claim 1, wherein the intracellular domain comprises an intracellular signal transduction region; optionally, the intracellular signal transduction region is derived from one or more of CD3ζ, CD3γ, CD3δ, CD3ε, CD5, CD22, CD79a, CD79b, FcRγ, FcRβ, CD66d, DAP10, DAP12 and Syk; further optionally, the intracellular signal transduction region is derived from CD3ζ; even further optionally, the amino acid sequence of the intracellular signal transduction region comprises the amino acid sequence as represented by SEQ ID NO: 23.

9. The bispecific chimeric antigen receptor according to claim 1, wherein the intracellular domain further comprises a costimulatory signal transduction region; optionally, the costimulatory signal transduction region is derived from one, two or more of CD2, CD3, CD7, CD27, CD28, CD30, CD40, CD83, CD244, 4-1BB, OX40, LFA-1, ICOS, LIGHT, NKG2C, NKG2D, DAP10, B7-H3 and MyD88; further optionally, the costimulatory signal transduction region is derived from CD28 or 4-1BB; even further optionally, the amino acid sequence of the costimulatory signal transduction region comprises the amino acid sequence as represented by SEQ ID NO: 24.

10. The bispecific chimeric antigen receptor according to any one of claims 1-9, further comprising a guiding peptide located at the N-terminus of the amino acid sequence of the chimeric antigen receptor; optionally, the guiding peptide is derived from CD8α; further optionally, the amino acid sequence of the guiding peptide comprises the amino acid sequence as represented by SEQ ID NO: 25.

11. The bispecific chimeric antigen receptor according to any one of claims 1-10, wherein the bispecific chimeric antigen receptor comprises the amino acid sequence as represented by SEQ ID NO: 28 or SEQ ID NO: 29.

12. An isolated nucleic acid molecule comprising a nucleotide sequence encoding the bispecific chimeric antigen receptor according to any one of claims 1-11;
optionally, the nucleotide sequence encoding the bispecific chimeric antigen receptor comprises:
1) a nucleotide sequence encoding a BCMA VH amino acid sequence as represented by SEQ ID NO: 13, which is represented by SEQ ID NO: 37; and a nucleotide sequence encoding a BCMA VL amino acid sequence as represented by SEQ ID NO: 14, which is represented by SEQ ID NO: 38; and/or
2) a nucleotide sequence encoding a CD19 VH amino acid sequence as represented by SEQ ID NO: 17, which is represented by SEQ ID NO: 39; and a nucleotide sequence encoding a CD19 VL amino acid sequence as represented by SEQ ID NO: 18, which is represented by SEQ ID NO: 40.

13. A vector comprising the isolated nucleic acid molecule according to claim 12;
optionally, the vector is an expression vector;
further optionally, the vector is a viral vector;
even further optionally, the vector is a lentiviral vector.

14. An engineered immune effector cell comprising the chimeric antigen receptor according to any one of claims 1-11, the isolated nucleic acid molecule according to claim 12 or the vector according to claim 13.

15. The engineered immune effector cell according to claim 14, wherein the engineered immune effector cell is selected from one or more of T lymphocyte, natural killer cell (NK cell), peripheral blood mononuclear cell (PBMC cell), pluripotent stem cell, T cell differentiated from pluripotent stem cell, NK cell differentiated from pluripotent stem cell, induced pluripotent stem cell (iPSC), T cell differentiated from induced pluripotent stem cell (iPSC-T), NK cell differentiated from induced pluripotent stem cell (iPSC-NK) and embryonic stem cell;
optionally, the engineered immune effector cell is a T lymphocyte;
further optionally, the source of the T lymphocyte is autologous T lymphocyte or allogeneic T lymphocyte.

16. A pharmaceutical composition comprising the engineered immune effector cell according to claim 14 or 15, and a pharmaceutically acceptable adjuvant; optionally, the pharmaceutically acceptable adjuvant includes a protective agent; optionally, the pharmaceutically acceptable adjuvant includes a cell cryopreservation solution.

17. The pharmaceutical composition according to claim 16, wherein the pharmaceutical composition is an intravenous injection.

18. Use of the chimeric antigen receptor according to any one of claims 1-11, the isolated nucleic acid molecule according to claim 12, the vector according to claim 13 or the engineered immune effector cell according to claim 14 or 15 in the preparation of a medicament, wherein the medicament is used for treating a disease or condition associated with the expression of BCMA.

19. The use according to claim 18, wherein the disease or condition associated with the expression of BCMA is cancer; optionally, the cancer is multiple myeloma; further optionally, the cancer is refractory or relapsed multiple myeloma.

20. The use according to claim 18, wherein the disease or condition associated with the expression of BCMA is an autoimmune disease; optionally, the autoimmune disease is selected from the group consisting of systemic lupus erythematosus, rheumatoid arthritis, idiopathic thrombocytopenic purpura, myasthenia gravis, and autoimmune hemolytic anemia.

21. A method for treating a disease or condition associated with the expression of BCMA, including the following steps: administering an effective amount of the engineered immune effector cell according to claim 14 or 15 or the pharmaceutical composition according to claim 16 or 17 to a subject having a need to treat a disease or condition associated with the expression of BCMA.

22. The method according to claim 21, wherein the disease or condition associated with the expression of BCMA is cancer; optionally, the cancer is multiple myeloma; further optionally, the cancer is refractory or relapsed multiple myeloma.

23. The method according to claim 21, wherein the disease or condition associated with the expression of BCMA is an autoimmune disease; optionally, the autoimmune disease is selected from the group consisting of systemic lupus erythematosus, rheumatoid arthritis, idiopathic thrombocytopenic purpura, myasthenia gravis, and autoimmune hemolytic anemia.

24. The method according to any one of claims 21-23, wherein the administration method is intravenous injection;
optionally, the administration method is to administer an effective amount of the engineered immune effector cell according to claim 14 or 15 or the pharmaceutical composition according to claim 16 or 17 to a subject in a single injection;
further optionally, the effective amount of the engineered immune effector cell according to claim 14 or 15 or the pharmaceutical composition according to claim 16 or 17 is at a dose of 1×10⁵ to 1×10⁷ cells/kg.

25. The engineered immune effector cell according to claim 14 or 15 or the pharmaceutical composition according to claim 16 or 17, for use in treating a disease or condition associated with the expression of BCMA.

26. The engineered immune effector cell or the pharmaceutical composition according to claim 25, wherein the disease or condition associated with the expression of BCMA is cancer; optionally, the cancer is multiple myeloma; further optionally, the cancer is refractory or relapsed multiple myeloma.

27. The engineered immune effector cell or the pharmaceutical composition according to claim 25, wherein the disease or condition associated with the expression of BCMA is an autoimmune disease; optionally, the autoimmune disease is selected from the group consisting of systemic lupus erythematosus, rheumatoid arthritis, idiopathic thrombocytopenic purpura, myasthenia gravis, and autoimmune hemolytic anemia.
